## Europäisches Patentamt
## European Patent Office
## Office européen des brevets

(19)

(11) Veröffentlichungsnummer: **0 112 435**
**B1**

(12)

# EUROPÄISCHE PATENTSCHRIFT

(45) Veröffentlichungstag der Patentschrift:
14.01.87

(51) Int. Cl.⁴: **A 61 F 2/36**

(21) Anmeldenummer: 83106198.9

(22) Anmeldetag: 19.06.83

(54) Femurkopfprothese.

(30) Priorität: 20.12.82 CH 7412/82

(43) Veröffentlichungstag der Anmeldung:
04.07.84 Patentblatt 84/27

(45) Bekanntmachung des Hinweises auf die Patenterteilung:
14.01.87 Patentblatt 87/3

(84) Benannte Vertragsstaaten:
AT DE FR GB IT

(56) Entgegenhaltungen:
DE-A-2 839 092
FR-A-2 438 468
US-A-4 068 324

(73) Patentinhaber: GEBRÜDER SULZER AKTIENGESELLSCHAFT, Zürcherstrasse 9, CH-8401 Winterthur (CH)

(72) Erfinder: Gries, Peter, Prof., Dr.- med., Meerfeldstrasse 69, D-6800 Mannheim (DE)

(74) Vertreter: Dipl.- Ing. H. Marsch Dipl.- Ing. K. Sparing Dipl.- Phys.Dr. W.H. Röhl Patentanwälte, Rethelstrasse 123, D-4000 Düsseldorf (DE)

EP 0 112 435 B1

## Beschreibung

Die Erfindung betrifft eine Femurkopfprothese, deren Kopf auf einem Prothesenhals sitzt, der seinerseits in einen in einem Knochenzementbett zu verankernden Verankerungsschaft übergeht, wobei der im Querschnitt im wesentlichen rechteckige Verankerungsschaft in einem stetig gekrümmten Bogen in ein in Richtung der Schaftachse verlaufendes, sich konisch verjüngendes, distales Ende einmündet.

Eine Prothese der genannten Art ist bekannt aus der CH-AS- 599 748; bei dieser Konstruktion hat der Schaft im proximalen Bereich einen T- oder L-förmigen Querschnitt, um die Druckverteilung der auf den Knochenzement wirkenden Belastung zu vergleichmässigen. Dem gleichen Ziel dient eine Konstruktion (US-A- 4,068,324), bei der der mediale Schaftbereich von proximal nach distal im Querschnitt stufenförmig verringert wird. Die Stufen haben die zusätzliche Aufgabe, den Knochenzement beim Einschlagen des Schaftes zu komprimieren.

Neben einer gleichmässigen Verteilung der Belastung auf den Knochenzement ist bekanntlich für einen festen Sitz eines mit Hilfe von Knochenzement verankerten Prothesenschaftes darüberhinaus entscheidend, dass vor allem die Umfangskomponente der bei der Polymerisation unvermeidbar auftretenden Schwindung des - im allgemeinen im wesentlichen aus Polymethylmethacrylat bestehenden - Knochenzementbettes nicht zu Hohlräumen zwischen Schaftoberfläche und Zement bzw. zwischen diesen und dem Knochengewebe führt; denn die "im Betrieb" der Prothese auftretenden Be- und Entlastungen führen zu Erweiterungen derartiger Hohlräume, die auf die Dauer ein Zerbröckeln des Zementbettes und/oder - infolge der dabei auftretenden Mikrobewegungen des Zementbettes relativ zu dem an ihm anliegenden Knochengewebe einen Abbau des Knochens und schliesslich Schaftlockerungen verursachen.

Weiterhin sind zur zementfreien Verankerung von Femurkopfprothesen im Querschnitt rechteckige Schäfte bekannt (DE-Al 25 17 702 und DE-B2 24 61 339), bei denen die laterale und die mediale Schmalseite stufenförmig ausgebildet und darüberhinaus auf den Breitseiten schräg zur Längsachse des Schaftes verlaufende Absätze vorgesehen sind. Diese Schäfte sind für eine Verankerung in einem Knochenzementbett nicht geeignet.

Aufgabe der Erfindung ist es, eine Schaftkonstruktion für einen mit Hilfe von Knochenzement zu verankernden Schaft einer Femurkopfprothese zu schaffen, deren Form die Gefahr der Bildung der erwähnten Hohlräume soweit wie möglich vermindert; selbstverständlich ist dabei auch die bekannte Zusatzforderung zu beachten, dass - um Reoperationen zu erleichtern - der Schaft aus dem Knochenzementbett herausziehbar sein muss, ohne dass dieses dabei zerstört werden muss. Diese Aufgabe wird nach der Erfindung dadurch gelöst, dass die Dicke des Verankerungsschaftes beiderseits und senkrecht zu einer zu den Breitseiten parallelen Mittelebene, im Querschnitt von medial nach lateral fortschreitend, stufenförmig zunächst bis zu einer maximalen Dicke erweitert und von dieser maximalen Dicke ebenso stufenförmig in mindestens einer Stufe zur lateralen Schmalseite hin wieder verringert ist.

Die stufenförmige Verbreiterung des Schaftes, an die sich, ebenfalls stufenförmig, nach lateral wieder eine Querschnittsverengung anschliesst, bewirkt, dass der den Schaft als geschlossener, peripherer Ring umschliessende Knochenzement, dessen lineare Schwindung etwa 2 % beträgt, sich an die abgerundeten Ecken und Kanten der Stufen besser anschmiegt und besonders infolge der Vielzahl der Ecken fester haftet als bei bisherigen Schaftformen. Ein zusätzlicher Vorteil der neuen Konstruktion besteht darin, dass durch die Stufenbildung die Oberfläche des Schaftes und damit die Grenzfläche Schaft/Knochenzement vergrössert wird, was bekanntlich ("Zeitschrift für Orthopädie", Heft 1, Februar 1974, Seite 27 - 33) die Stabilität einer Prothesenverankerung verbessert.

Eine in peripherer Richtung weitgehend gleichmässige Schwindung und damit Haftung des Zements an dem Schaft erreicht man, wenn der Bereich der maximalen Dicke etwa symmetrisch zur in der Mittelebene von medial nach lateral gemessenen Mitte der Breitseiten des Schaftes angeordnet ist. Weiterhin ergibt sich ein verbesserter Sitz des Schaftes in dem Knochenzementbett, wenn sich die Kanten oder Stufen bei Biegebelastungen auf dem Zementbett abstützen können; dazu ist es vorteilhaft, wenn mindestens medialseitig der Maximaldicke die die Stufen erzeugenden Kanten im Bereich des stetig gekrümmten Bogen ebenfalls nach medial gekrümmt sind.

Im folgenden wird die Erfindung anhand von zwei Ausführungsbeispielen im Zusammenhang mit der Zeichnung näher erläutert.

Fig. 1 zeigt eine Ansicht eines erfindungsgemäss ausgebildeten Prothesenschaftes;

Fig. 2 ist ein Schnitt II-II von Fig. 1;

Fig. 3 gibt eine zweite Ausführungsform der Erfindung wieder, von der

Fig. 4 der Schnitt IV-IV ist.

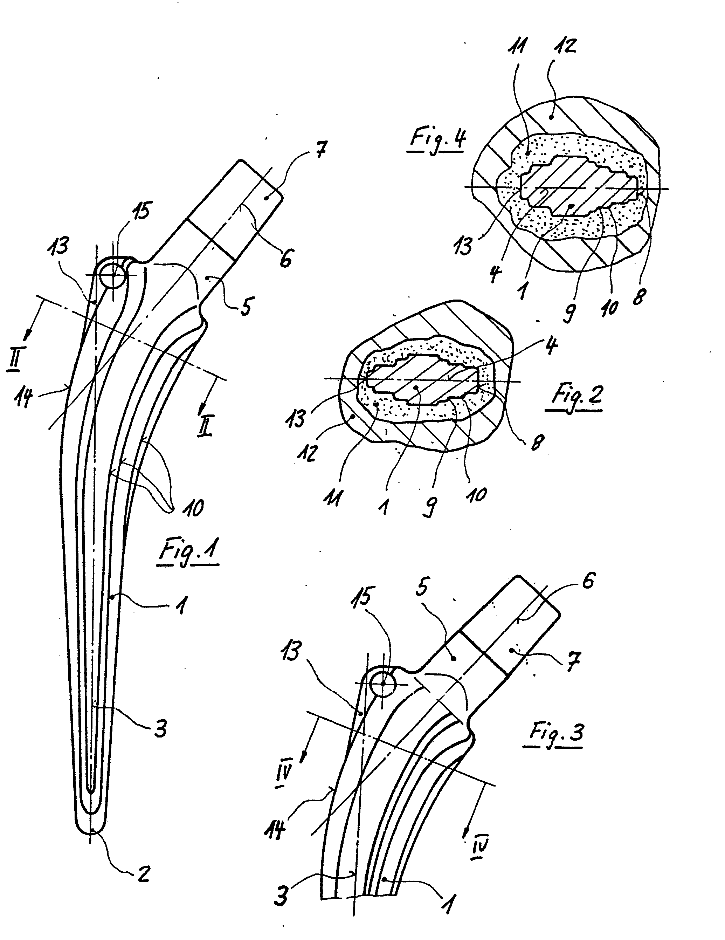

Der Schaft 1 für eine Femurkopfprothese erweitert sich von seinem distalen Ende 2 aus zunächst symmetrisch zu einer seine Längsachse 3 enthaltenden Mittelebene; etwa auf der halben Schafthöhe - gemessen von "Spitze" zu "Spitze" entlang der Längsachse 3 - geht der Konus in einen Bogen über, wobei die Erweiterung der Schaftbreite - gemessen in der Symmetrie- oder Mittelebene 4 des Querschnitts (Fig. 2) - zum Prothesenhals 5 hin sich stetig fortsetzt. Der Prothesenhals 5, dessen Achse 6 mit der Längsachse 3 des Schaftes 1 einen Winkel von etwa 45 bildet, trägt einen konischen Zapfen 7 zur Befestigung eines nicht gezeigten Gelenkkopfes.

Erfindungsgemäss ist die Dicke des Schaftes 1 - gemessen senkrecht zur Mittelebene 4 von Fig. 2 - ausgehend von seiner medialen Schmalseite 8 (Fig. 2) bis zu einer Maximaldicke nach beiden Seiten symmetrisch zur Mittelebene 4 stufenförmig vergrössert, wodurch sich Ecken 9 und Kanten 10 ergeben, die zur Vermeidung von Belastungsspitzen und Ermüdungsrissen abgerundet sind.

Vom Bereich der Maximaldicke verringert sich der Querschnitt des Schaftes 1 in lateraler Richtung wieder in einer oder mehreren Stufen. Anzahl und Tiefe der Stufen können dabei von der Maximaldicke aus gesehen nach medial und lateral gleich (Fig. 1 und 2) oder verschieden sein, wie Fig. 3 und 4 erkennen lassen.

Weiterhin ist es zweckmässig, wenn die das reliefartige Profil erzeugenden Kanten 10 mindestens annähernd parallel zur Aussenbegrenzung des Schaftes 1 verlaufen; dabei sind mindestens im Bereich des stetig gekrümmten Bogens die Kanten 10 zur zusätzlichen Abstützung der Prothese auf dem Knochenzementbett 11, das den vom spongiosen Gewebe geräumten kortikalen Mantel 12 (Fig. 2 und 4) des Femurknochens ausfüllt, nach medial gekrümmt.

In einem flügelartigen Ansatz 13 ist am proximalen Schaftende auf der lateralen Seite, d.h. im Bereich des Uebergangs der lateralen Schmalseite 14 in eine zum Prothesenhals 5 führende Schulter in an sich bekannter Weise eine Bohrung 15 vorgesehen, in die ein nicht gezeigtes Ausziehinstrument eingreift, falls aus irgendwelchen Gründen eine Reoperation des Prothesenschaftes erforderlich ist.

## Patentansprüche

1. Femurkopfprothese, deren Kopf auf einem Prothesenhals (5) sitzt, der seinerseits in einen in einem Knochenzementbett (11) zu verankernden Verankerungsschaft (1) übergeht, wobei der im Querschnitt im wesentlichen rechteckige Verankerungsschaft in einem stetig gekrümmten Bogen in ein in Richtung der Schaftachse (3) verlaufendes, sich konisch verjüngendes, distales Ende (2) einmündet, dadurch gekennzeichnet, dass die Dicke des Verankerungsschaftes (1) beiderseits und senkrecht zu einer zu den Breitseiten parallelen Mittelebene (4), im Querschnitt von medial nach lateral fortschreitend, stufenförmig zunächst bis zu einer maximalen Dicke erweitert und von dieser maximalen Dicke ebenso stufenförmig in mindestens einer Stufe zur lateralen Schmalseite (14) hin wieder verringert ist.

2. Femurkopfprothese nach Anspruch 1, dadurch gekennzeichnet, dass der Bereich der maximalen Dicke etwa symmetrisch zur in der Mittelebene (4) von medial nach lateral gemessenen Mitte der Breitseiten des Verankerungsschaftes (1) angeordnet ist.

3. Femurkopfprothese nach Anspruch 1, dadurch gekennzeichnet, dass medialseitig der maximalen Schaftdicke die die Stufen erzeugenden Kanten (10) im Bereich des stetig gekrümmten Bogens ebenfalls nach medial gekrümmt sind.

## Claims

1. A femoral head prosthesis whose head is disposed on a prosthetic neck (5) merging into an anchorage stem (1) anchorable in a bone cement bed (11), the substantially rectangular section stem extending in a continuous curvature into a conically narrowing distal end (2) which extends in the direction of the stem axis (3), characterised in that the thickness of the stem (1) widens stepwise initially to a maximum thickness on both sides and perpendicularly to a central plane (4) parallel to the wide sides, progressing in cross-section from medial to lateral, and decreases stepwise from this maximum thickness in at least one step to the lateral narrow side (14).

2. A prosthetic femoral head according to claim 1, characterised in that the zone of maximum thickness is disposed substantially symmetrically of the centre of the wide sides of the stem (1), such centre being measured in the centre-plane (4) from medial to lateral.

3. A prosthetic femoral head according to claim 1, characterised in that on the medial side of maximum stem thickness the edges (10) producing the steps are also curved in the medial direction in the region of the continuous curvature.

## Revendications

1. Prothèse pour tête de fémur, dont la tête repose sur un col (5) de prothèse se prolongeant elle-même par une tige d'ancrage (1) devant être ancrée dans un lit d'ostéociment (11), la tige d'ancrage de section sensiblement rectangulaire débouchant, par un arc à courbure constante, dans une extrémité distale (2) qui s'étend dans la direction de l'axe (3) de la tige et présente un rétrécissement conique de section, caractérisée par le fait que l'épaisseur de la tige d'ancrage (1) augmente tout d'abord par paliers jusqu'à une épaisseur maximale, de part et d'autre et perpendiculairement à un plan médian (4) parallèle aux côtés larges, avec progression de section de la région médiale à la région latérale, puis diminue également par paliers à partir de cette épaisseur maximale en direction de la face étroite latérale (14), d'au moins un palier.

2. Prothèse pour tête de fémur selon la revendication 1, caractérisée par le fait que la région d'épaisseur maximale est disposée sensiblement symétriquement par rapport au milieu des côtés larges de la tige d'ancrage (1), mesuré dans le plan médian (4), de la région médiale à la région latérale.

3. Prothèse pour tête de fémur selon la revendication 1, caractérisée par le fait que, du côté médial de l'épaisseur maximale de la tige, les arêtes (10) produisant les échelons accusent également une courbure vers la région médiale, au voisinage de l'arc à courbure constante.

0 112 435

Fig. 4

Fig. 2

Fig. 1

Fig. 3